# EUROPEAN PATENT APPLICATION

(11) **EP 1 779 849 A1**
(43) Date of publication of application: **02.05.2007**
(21) Application number: 05110163.2
(22) Date of filing: 28.10.2005
(51) Int. Cl.: A61K 31/365, A61K 31/7048, A61K 38/15, A61P 31/00, A61K 31/4015, A61K 31/4166

(54) **V-ATPase inhibitors for the treatment of septic shock**

(71) Applicant: NIKEM RESEARCH S.R.L., 20021 Baranzate di Bollate (IT)
(72) Inventor: Farina, Carlo, 20021, BARANZATE DI BOLLATE (IT); Constantin, Gabriela, 37020, S. FLORIANO (IT); Laudanna, Carlo, 37020, S. FLORIANO (IT); Misiano, Paola, 20021, BARANZATE DI BOLLATE (IT)
(74) Representative: Gerli, Paolo

(57) **Abstract**

The use of vacuolar ATPase inhibitors in the prevention and/or treatment of septic shock is herein described. Vacuolar ATPase inhibitors are selected among products of natural, semi-synthetic or synthetic origin; more preferred are inhibitors based on a 2- and/or 3-substituted indole, azaindole or benzoimidazole structure.

## Description

### FIELD OF THE INVENTION

The present invention relates to the filed of the medical treatment of septic shock.

### STATE OF THE ART

In clinic, sepsis is defined as the systemic host response to infection with a "Systemic Inflammatory Response Syndrome" (SIRS) plus a documented infection. Severe sepsis is defined as sepsis plus end-organ dysfunction or hypo-perfusion, and septic shock is as sepsis with hypotension, and evidence of global inadequate tissue and organ perfusion.

SIRS, sepsis, and septic shock are most commonly associated with bacterial infection. Bacteremia, i.e. the presence of viable bacterial within blood, if present, may be primary (without an identifiable focus of infection) or, more often, secondary (with an intravascular or extravascular focus of infection).

The most common cause of septic shock are gram-negative bacteria, followed by gram-positive bacteria, fungi and, less often, viruses. In septic shock due to bacterial infection, circulatory insufficiency occurs when bacterial products (endotoxins, esotoxins) interact with host cells and serum proteins to initiate a series of reactions that ultimately may lead to cell injury, systemic failure and death. These bacterial products themselves are harmful, and the widespread and unregulated host response to these substances results in the elaboration of an extensive array of pro-inflammatory chemical mediators that lead to further cell damage.Bacteria products such as the lypopolysaccharide (LPS, often called endotoxin), are major responsible of the systemic inflammatory response. LPS behaves as a pleiotropic factor by acting directly on cells, such as endothelial cells, monocytes, neutrophils and macrophages, and facilitates the recruitment of leukocyte into the tissues. Then, these cells start to release a variety of pro-inflammatory molecules, such as cytokines (TNF, IL-1, IL-2, IL-6, IL-8) and a number of arachidonic acid derivative (PAF, PGE, LTB4) which amplify and maintain the systemic proinflammatory reaction. Furthermore, LPS can activate the complement and pro-coagulation pathways in the plasma, thus leading to further accumulation of pro-inflammatory mediators. This may cause a generalized vasodilatation accompanied by intravascular coagulation and increased vessel permeability. Overall, during a septic shock the massive and very rapid accumulation of soluble mediators of inflammation in the blood and peripheral tissues determine a generalized inflammation and a reduced perfusion with severe damage of several vital organs, such as lung, liver, kidney and heart.

Sepsis occurs in about 2% of total hospitalization. Septic shock develops in fewer than half of patients with bacteremia. It occurs in about 50% of patients with gram-negative bacteremia and in about 20% of patients with *Staphylococcus aureus* bacteremia.

Significant complications from sepsis include central nervous system dysfunction (19%), adult respiratory distress syndrome (ARDS) (2-8%), liver failure (12%), acute renal failure (ARF) (9-23%), and disseminated intravascular coagulation (DIC) (8-18%). In septic shock, ARDS has been observed in about 18% of cases, DIC in about 38% and renal failure in about 50%.

The mortality in sepsis varies according to the degree of pathologic derangement and the presence of a documented infection: in SIRS has been reported to be about 7%, in sepsis is about 16-20%, and in septic shock is about 45-60%. Normally, more than 50-60 % of patients with septic shock die within 30 days, and another 10% die in the next 6 months as a consequence of generalized damages of vital organs.

Septic shock is an acute syndrome characterized, as every shock, by failure of the cardio-circulatory system followed by organ hypo-anoxia. Treatment must be aggressive to rapidly sustain blood pressure, circulation and organ perfusion. Antibiotic must be used to block the progression of infection, anti-coagulant treatment is also necessary to prevent embolic damages, and anti-adhesive treatment is essential to prevent leukocyte recruitment and activation. Overall, the pharmacological treatment of septic shock is always very difficult and poorly efficacious, and this area has great unmet medical needs.

In septic shock, and especially in endotoxic shock, rapid leukocyte activation is a central pathogenetic event. LPS may trigger proadhesive mechanisms in the endothelial cells thus leading to integrin-mediated leukocytes adhesion to the blood vessels. Integrin and chemoattractants may, in turn, activate leukocytes to release oxygen free radicals, to secrete acute phase proteins and arachidonic acid metabolites. Moreover, transmigrated leukocytes start to release cytokine that further affect endothelial cells, thus maintaining and amplifying the pathogenetic process. Release of free radicals and protein secretion are responsible of tissue damage and amplify the inflammation. Thus, beside the damage due to reduced tissue perfusion, in septic shock leukocyte activation determines damage due to a generalized tissue inflammation.

Complex signal transduction mechanisms control the different aspects of leukocyte activation, including specific signaling pathways controlling integrin activation, chemotaxis and the activation of NADPH-oxidase, the enzyme responsible of oxygen free radical production.

The vacuolar proton translocating ATPases (V-ATPases) are widely expressed in eucaryotic cell endomembranes where protons are pumped into the organelle lumen using the energy released by ATP hydrolysis. The active transport of protons across animal cell plasma membranes appears to be restricted to cells that are specialized in proton secretion such as macrophages (J.Biol.Chem., 265,7645, 1990)*,* and osteoclasts (J.Cell.Biol., 1987, 105, 1637). In these cells, V-ATPase actively secretes protons from the cells and establishes an acidic extracellular environment useful respectively for bacteria phagocytosis, urinary acidification, and bone resorption. The inhibition of V-ATPase has recently been identified as a possible therapeutic target. In particular, V-ATPase inhibitor have been described for use in preventing bone loss and inhibiting bone resorption, thereby being useful in the treatment of osteoporosis and other conditions related to osteoclast hyperactivity *(*Acta Physiol.Scand., , 163(suppl.), 195, 1998*.;* J.Clin.Invest., , 106, 309, 2000*).* A number of V-ATPase inhibitors useful for the treatment of osteoclast-related disorders and/or tumours have been the subject of a number of the patent applications US2002099080, WO9801443, WO0100587, WO0102388, PCT/EP2005/051908, PCT/EP2005/051910.

### SUMMARY

We have now discovered that V-ATPase inhibitors have useful properties for the treatment of septic shock, as well as immunomodulatory effects on cells of innate and acquired immunity, together with anti-inflammatory properties.

In the present application we describe a potent inhibitory activity of these compounds on adhesion, chemotaxis and free radicals release of immuno-competent cells; these data support the use of said inhibitors in the treatment and/or prevention of septic shock, as well as of acute and chronic inflammatory diseases and autoimmune diseases.

### DESCRIPTION OF THE FIGURES

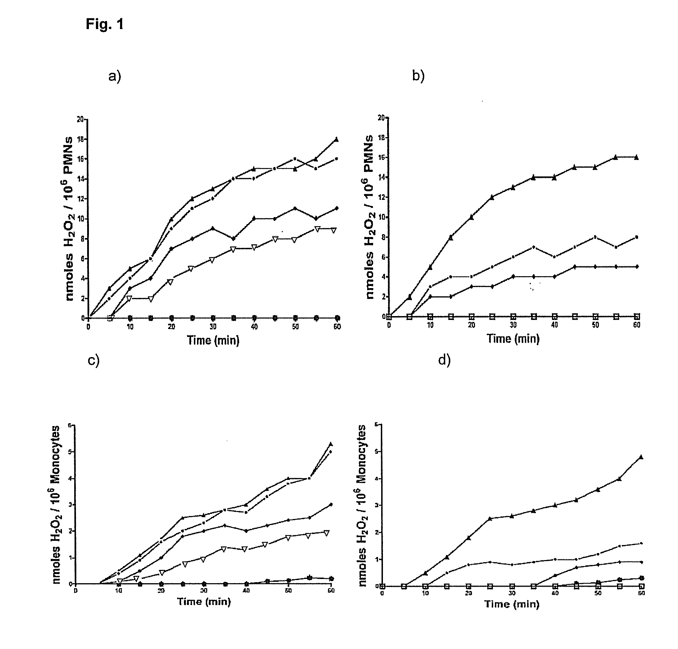

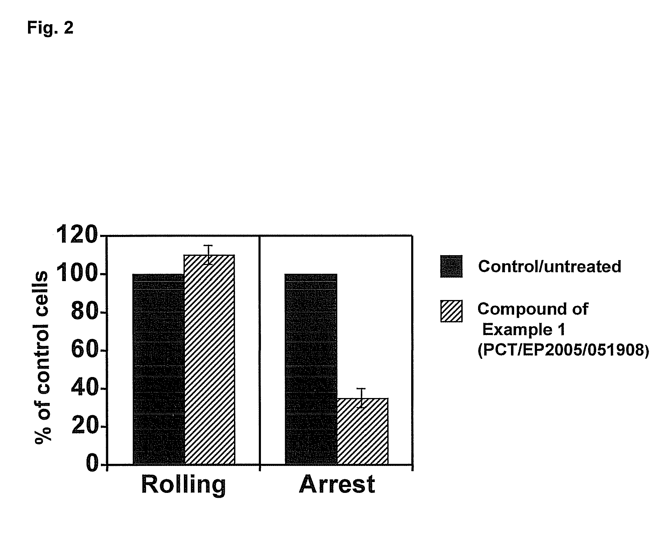

**Figure 1**: Effect of V-ATPase inhibitors on NADPH-oxidase activation
   **a)** Effect of Compound of Example 1 (PCT/EP2005/051908) in PMNs:
      -▲- :Control with PMA;
      -•-:10 µM Compound of Example 1 with PMA;
      -◆-:50 µM Compound of Example 1 with PMA;
      - - : 100 µM Compound of Example 1 with PMA;
      - 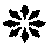 - Control without PMA
   **b)** Effect of Compound of Example 2 (PCT/EP2005/051910) in PMNs:
      -▲- :Control with PMA;
      -●- : µM Compound of Example 2 with PMA;
      -◆- :5 µM Compound of Example 2 with PMA;
      -□- :10 µM Compound of Example 2 with PMA, and control without PMA
      (n.b.: both these curves overlap in figure 1b)
   **c)** Effect of Compound of Example 1 (PCT/EP2005/051908) in Monocytes:
      -▲-: Control with PMA;
      -•-:10 µM Compound of Example 1 with PMA;
      -◆-:50 µM Compound of Example 1 with PMA;
      - -:100 µM Compound of Example 1 with PMA;
      - 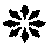 - : Control without PMA
   **d)** Effect of Compound of Example 2 (PCT/EP2005/051910) in Monocytes:
      -▲-: Control with PMA;
      -•- :1 µM Compound of Example 2 with PMA;
      -◆-:5 µM Compound of Example 2 with PMA;
      -□-:10 µM Compound of Example 2 with PMA;
      - 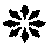 - : Control without PMA;
      (n.b.: the curves -◆-, -□- and - 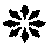 - partly overlap in figure 1d.)
**Figure 2**: Effect of Compound of Example 1 (PCT/EP2005/051908) on lymphocyte recruitment in inflamed brain microcirculation evaluated by intravital microscopy technique:
   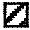 Control/untreated
   ■ Compound of the Example 1 (PCT/EP2005/051908)

### DETAILED DESCRIPTION OF THE INVENTION

Object of the present invention is the use of one or more V-ATPase inhibitors in the preparation of a medicament useful for preventing and/or treating septic shock.

The invention also comprises a method for preventing and/or treating septic shock characterised by the administration of a pharmaceutically effective amount of a compound of formula (I) to a patient in need thereof.

The prevention and/or treatment of inflammatory and/or autoimmune diseases by use of said V-ATPase inhibitors is object of co-pending application in the name of the present Applicant.

V-ATPase inhibitors are a recognised class of compounds, as reviewed in *Curr.Pharm.Design"8,2033-2048, 2002.* In general, for V-ATPase inhibitor is meant a compound which, when assayed according to James I.E. et al. - J. Bone Min. Res. 14, 1562-1569, 1999 and Nadler, G. et al.-Bioorg. Med. Chem. Letters 8, 3621-3626, 1998*,* shows at least 50% Bafilomycin-sensitive inhibitory activity at 5 µM.

V-ATPase inhibitors may be of natural origin, semi-synthetic or entirely synthetic.

Non limiting examples of V-ATPase inhibitors of natural origin are the macrolides bafilomycins, concanamycins, depsipeptide mycotoxins derived from the fungi *Metharisium anisopliae,* destruxin, lobatamides, salicylihalamides, oximidines; non-limiting examples of semi-synthetic derivatives are the sulphonamide derivatives of bafilomycins, 7,21-O-disubstituted bafilomycins, 2-methoxy-2,4-pentadienoic esters of bafilomycins, etc.; non-limiting examples of entirely synthetic V-ATPase inhibitors are N-ethylmaleimide, 7-chloro-4-nitrobenzo-2-oxa-1,3-diazole, and derivatives thereof, the compounds WY 47766, SB242784, aminoquinoline derivatives like the compound FR167356. A particularly preferred class of V-ATPase inhibitors is that of 2- and/or 3- substituted indoles, azaindoles, benzimidazoles; within this class, even more preferred are the inhibitors disclosed in the following patent applications, all of which are incorporated herein by reference.
(i) US2002099080, describing compounds of formula 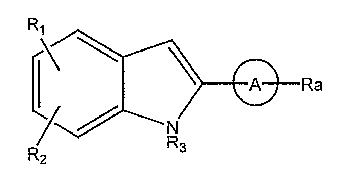 wherein A, R1-3, Ra have the meanings therein indicated
(ii) WO9801443, describing compounds of formula 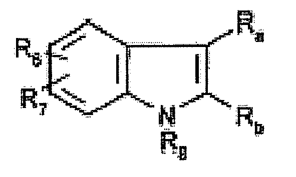 wherein R6-8, Ra, Rb have the meanings therein indicated.
(iii) WO0100587, describing compounds of formula: 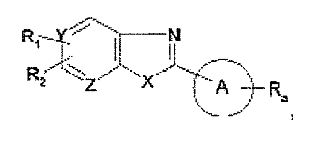 wherein R1, R2, Ra, A, X,Y,Z have the meanings therein indicated.
(iv) WO0102388, describing compounds of formula 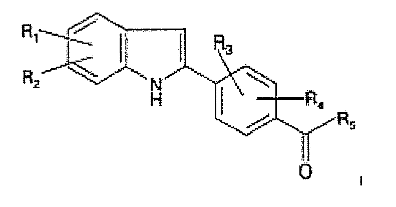 wherein R1-R5 have the meanings therein indicated.
(v) PCT/EP2005/051908, describing compounds of formula: 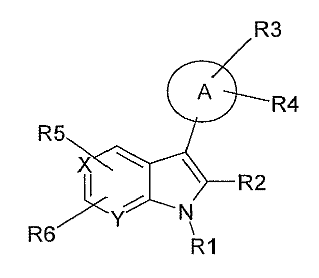 wherein:
   R1 is chosen from H, alkyl, arylalkyl, hydroxyalkyl, alkoxyalkyl, aminoalkyl, alkylaminoalkyl, dialkylaminoalkyl, alkylCOOalkyl, alkoxycarbonylalkyl, aminocarbonylalkyl, alkylaminocarbonylalkyl, dialkylaminocarbonylalkyl, alkylCONalkyl, cyanoalkyl, or a group R'R"Nalkyl, in which R' and R", together with the nitrogen atom to which they are attached, may form a 5, 6 or 7 membered ring, optionally containing a heteroatom chosen from O, S and N, and where said N atom may be substituted by alkyl;
   R2 is chosen from alkyl, alkenyl, aryl, heterocyclyl optionally substituted by alkyl or aryl, acid, ester, amide, nitrile, aminoalkyl, alkylaminoalkyl, dialkylaminoalkyl, hydroxyalkyl, CH₂NHCOCH₃, CONHSO₂CH₃, alkoxycarbonylalkyl, alkoxycarbonylalkenyl; or R1 and R2 together form a 5, 6 or 7 membered ring containing optionally a heteroatom chosen from O, S, N and containing optionally a carbonyl function which can be attached to any carbon atom of said ring, and where said N atom may be substituted by alkyl, aryl, arylalkyl, heteroaryl, alkylsulfonyl, arylsulfonyl, alkylcarbonyl, arylcarbonyl, alkylaminocarbonyl, arylaminocarbonyl;
   R3, R4, R5, R6 each independently represent H, alkyl, alkoxy, hydroxy, halogen, trifluoromethyl, trifluoromethyloxy;
   X and Y each independently represent carbon or nitrogen;
   A is chosen from a phenyl or a heterocyclic ring with 5 or 6 members containing up to two heteroatoms chosen from nitrogen, oxygen and sulfur.
(vi) PCT/EP2005/051910 describing compounds of formula: 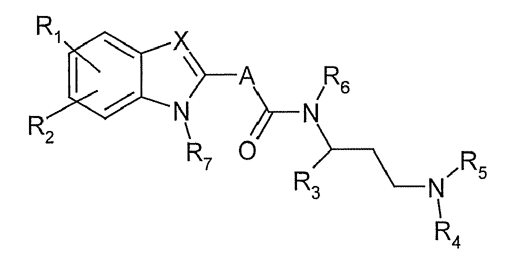 wherein:
   X is chosen from -CH- or -N-;
   A is chosen from the groups: 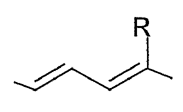 R= alkoxy, hydroxyalkoxy 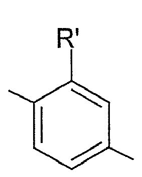 R'= hydroxy, alkoxy, hydroxyalkoxy, halogen
   R1 and R2, are each independently chosen from H, halogen and alkoxy;
   R3 and R4 are each independently chosen from H, alkyl, or R3 and R4, together with the atoms to which they are attached, form a 6, 7 or 8 membered heterocycle containing an atom of nitrogen, optionally substituted by one or more alkyl groups;
   R5 is chosen from H, alkyl, hydroxyalkyl, alkoxyalkyl, carboxyalkyl, aminoalkyl, optionally substituted aryl or arylalkyl, optionally substituted heterocyclyl or heterocyclylalkyl, or R5 and R4, together with the nitrogen atom to which they are attached, form an optionally substituted 5-8 membered heterocyclic ring containing up to 2 heteroatoms chosen from N, O and S;
   R6 and R7 are independently chosen from H and alkyl.

The present V-ATPase inhibitors can be suitably used in the treatment of septic shock. They can be administered as such or preferably as in the form of pharmaceutical compositions in the presence of suitable pharmaceutical excipients. The dosage units of these pharmaceutical compositions may contain said inhibitors in a quantity between 1 and 1000 mg. The above compositions and dosage units are adapted to deliver to the patients effective amounts of V-ATPase inhibitor. Said inhibitors are generally active within a dosage range comprised between 0.01 and 100 mg/Kg.

The V-ATPase inhibitors can be administered alone or in association with further drugs suitable for joint therapy with antishock drugs; these further drugs are selected according to rules well-known in the medical field.

The pharmaceutical compositions of the invention can be adapted for the various administration routes, and can be provided for example in the form of injectable solutions, solutions for infusion, solutions for inhalation, suspensions, emulsions, syrups, elixirs, drops, suppositories, possibly coated pills, hard or soft capsules, microcapsules, granules, dispersible powders etc.

The excipients contained in these compositions are those commonly used in pharmaceutical technology, and can be used in the manner and quantity commonly known to the expert of the art.

Solid administration forms, such as pills and capsules for oral administration, are normally supplied in dosage units. They contain conventional excipients such as binders, fillers, diluents, tabletting agents, lubricants, detergents, disintegrants, colorants and wetting agents and can be coated in accordance with methods well known in the art.

The fillers include for example cellulose, mannitol, lactose and similar agents. The disintegrants include starch, polyvinylpyrrolidone and starch derivatives such as sodium starch glycolate; the lubricants include, for example, magnesium stearate; the wetting agents include for example sodium lauryl sulfate.

These solid oral compositions can be prepared with conventional mixing, filling or tabletting methods. The mixing operations can be repeated to disperse the active agent in compositions containing large quantities of fillers. These are conventional operations.

The liquid preparations can be provided as such or in the form of a dry product to be reconstituted with water or with a suitable carrier at the time of use. These liquid preparations can contain conventional additives such as suspending agents, for example sorbitol, syrup, methylcellulose, gelatin, hydroxyethylcellulose, carboxymethylcellulose, aluminium stearate gel or hydrogenated edible fats, emulsifying agents, for example lecithin, sorbitan monooleate, or acacia; non aqueous carriers (which can include edible oil) for example almond oil, fractionated coconut oil, oily esters such a glycerin esters, propylene glycol or ethyl alcohol; preservatives, for example methyl or propyl p-hydroxybenzoate or sorbic acid and if desired, conventional flavours or colorants.

The oral formulations also include sustained release conventional formulations, such as enteric coated pills or granules.

For parenteral administration, fluid dosage units can be prepared containing the compound and a sterile carrier. The compound, depending on the carrier and concentration, can be suspended or dissolved. The parenteral solutions are normally prepared by dissolving the compound in a carrier and sterilizing by filtration, before filling suitable vials or ampoules and sealing. Adjuvants such as local anaesthetics, preservatives and buffering agents can be advantageously dissolved in the carrier. In order to increase stability, the composition can be frozen after filling the vial and the water removed under vacuum.

The parenteral suspensions are prepared essentially in the same way, with the difference that the compound can be suspended rather than dissolved in the carrier, and can be sterilized by exposure to ethylene oxide prior to being suspended in the sterile carrier. A surfactant or humectant can be advantageously included in the composition to facilitate uniform distribution of the compound of the invention.

As is the common practise, the compositions are normally accompanied by written or printed instructions, for use in the treatment concerned.

The invention is now illustrated by the following non-limiting examples.

### EXPERIMENTAL PART

### Methods

### In vitro studies

### Chemokine-induced adhesion in PMNs, lymphocytes and monocytes

Adhesion was performed on purified integrin ligands and was evaluated as reported in Constantin G. et al., Immunity, 16, 759-769, 2000*.* Briefly, primary naïve lymphocytes , polymorphonuclear neutrophils (PMNs) or monocytes were isolated form healthy donors. In other experiments, murine autoreactive T cells were obtained from SJL mice immunized with PLP139-151 as described (Piccio L. et al., J. Immunol. 168, 1940-1949, 2002). Cells were resuspended at 4x10⁶/ml in PBS, CaCl₂, MgCl₂ 1 mM, 10% FCS, pH 7.2. Adhesion assays were performed on eighteen well glass slides coated overnight at 4°C with ligands for CR3 (fibrinogen) or LFA-1 (ICAM-1). 20 µl of cell suspension were added to the wells and stimulated at 37°C with 5 µl of agonists prior to washing, fixation and computer-assisted enumeration of bound cells.

Integrin activation was measured as induction of rapid adhesion triggered by the classical chemoattractant N-formyl-L-methionyl-L-leucylphenylalanine (fMLP) (100 nM) or by the CC chemokine MIP3β (CCL19, 1 µM). PMNs and monocytes were triggered to adhere to fibrinogen (CR3 ligand); naive human lymphocytes or murine encephalitogenic T cells were triggered to adhere to ICAM-1 (LFA-1 ligand). Triggered adhesion was evaluated after 2 min of stimulation under static conditions. The cells were pretreated at 37°C for 30-60 min with the indicated concentrations of the test compound. Results are expressed as percentage of inhibition over control (= 100%) and are the mean of three experiments in the case of human leukocytes. Results are expressed as mean counts and are the mean counts ± S.D of three experiments and are the mean of three experiments in the case of murine encephalitogenic lymphocytes.

### Spontaneous adhesion of encephalitogenic T cells

Spontaneous adhesion of encephalitogenic lymphocytes was measured in adhesion assays on purified ICAM-1 and VCAM-1 as previously described (Constantin G. et al., J. Immunol. 162, 1144-1149, 1999). Autoreactive T cells were obtained from SJL mice immunized with PLP139-151 as described *(*Piccio L. et al., J. Immunol. 168, 1940-1949, 2002). Lymphocytes were treated with test compound at concentrations between 5-100µM for 1 h, while control/untreated cells were treated with vehicle (DMSO). Results are expressed as percentage of inhibition over control (= 100%) ± S.D and are the mean of three-four experiments.

### Chemotaxis assay

PMNs and monocytes migration was assessed using 1 or 3-µm pore size transwells (Bio-Coat Becton-Dickinson), while lymphocytes by using 5-µm pore size transwells.

Cells were in RPMI1640, containing 10% heat-inactivated Fetal Calf Serum (FCS) and 20 mM HEPES, pH 7.3, at 2x10⁶ /ml. 100 µl of cell suspension were added to the top well, and 600 µl of medium, containing chemoattractants and test compound, were added to the bottom well. Migration was evaluated after 2h for PMNs and monocytes and after 4h for lymphocytes. fMLP 10 nM (PMNs and monocytes) and MIP-3β 100 nM (lymphocytes) were utilized as chemoattractants. After fixation with 1.5% glutaraldehyde, migrated cells were counted by fluorescence-activated celll sorting using polystyrene beads (Polyscience) as an internal standard *(*Campbell J.J. et al. J. Cell. Biol. 134, 255-266, 1996*;* Laudanna C. et al. J. Biol. Chem. 273,46, 30306-30315, 1998*).*

Results are expressed as percentage of inhibition over control (= 100%), and are the mean of three experiments.

### Respiratory burst (H₂O₂ assay)

NADPH-oxidase activation was measured as induction of H₂O₂ release (derived from dismutation of O₂⁻) triggered in PMNs and monocytes by PMA (200 ng/ml).

Briefly, PMNs and monocytes were pretreated at 37°C for 30-60 min with test compound at different concentrations, and stimulated with phorbol 12-myristate 13-acetate (PMA, 200 ng/ml) dissolved in 0.5 ml of a standard reaction mixture. Release of H₂O₂ was evalauted in real time at time points of 5 min by using a microplate fluorescence reader (Perkin-Elmer). Results are expressed as nmoles of H₂O₂ released over time by 10⁶ cells. Shown are dose-response curves of inhibitory effect on H₂O₂ release over 60 min.

### In-vivo studies

### Intravital microscopy

Murine encephalitogenic T lymphocytes were labeled with either green CMFDA(5-chloromethylfluorescein diacetate; Molecular Probes) or orange CMTMR (5-(and-6)-(((4-chloromethyl)benzoyl)amino)tetramethylrhodamine) (Molecular Probes). SJL young females (Harlan-Nossan, Udine, Italy) were housed and usedaccording to current European Community rules. In order to inflame brain endothelium, mice were injected intraperitoneally with 12 µg lipopolysaccharide (LPS) *(Escherichia coli* 026:B6; Sigma) 5 to 6 hours before the intravital experiment was started.

Animals were anesthetized, and a heparinized PE-10 catheter (Intramedic Clay Adams, Sparks, MD) was inserted into the right common carotid artery toward the brain. To exclude noncerebral vessels from the analysis, the right external carotid artery and the pterygopalatine artery, a branch of the internal carotid, were ligated *(*Piccio L. et al. J. Immunol. 168, 1940-1949, 2002*).* The preparation was placed on an Olympus BX50WI microscope, and a water-immersion objective with long focal distance (Olympus Achroplan;focal distance, 3.3 mm; numeric aperture, 0.5°) was used. Blood vessels were visualized through the bone by using fluorescent dextrans. Fluorescence-labeled cells (2 x 10⁶ )/condition were slowly injected into the carotid artery through a digital pump. Images were visualized by using a silicon-intensified target videocamera (VE-1000 SIT; Dage MTI, Michigan City, IN) and a Sony SSM-125CE monitor and were recorded using a digital videocassette recorder (NV-DV10000; Panasonic).

Video analysis was performed by playback of digital videotapes. Vessel diameter (D), hemodynamic parameters, and the velocities of rolling were determined by using a personal computer-based system. The velocities of 20 or more consecutive freely flowing cells per venule were calculated.

From the velocity of the fastest cell in each venule (Vfast), we calculated the
mean blood flow velocities (Vₘ): Vₘ = V_{fast}/(2 -ε² ). Wall shear rate (WSR) was calculated from WSR= 8 x Vₘ/D (s⁻1), and wall shear stress (WSS) acting on rolling cells was approximated by WSR x 0.025 (dyne/cm²), assuming a blood viscosity of 0.025 Poise. Lymphocytes that remained stationary on the venular wall for 30 seconds or longer were considered adherent. At least 140 consecutive cells per venule were examined. Rolling and firm arrest fractions were determined as the percentages of cells that rolled or firmly arrested within a given venule in the total number of cells that entered that venule during the same period.

Lymphocytes were treated with DMSO (control cells) or with 50□M of test compound for 1 h before cell injection into the carotid artery for intravital microscopy experiments.

### Results

### In vitro studies

### Effect of V-ATPase inhibitors on rapid integrin activation and chemotaxis in human leukocytes

Integrin activation was measured as induction of rapid adhesion triggered by the "classical" chemoattractant fMLP (100 nM) or by the CC chemokine MIP3□ (CCL19, 1 µM). PMNs were triggered to adhere to fibrinogen (CR3 ligand); lymphocytes were triggered to adhere to ICAM-1 (LFA-1 ligand).

All the V-ATPase inhibitors (Table 1) were able to inhibit rapid adhesion in both PMNs and lymphocytes, in a concentration-related manner. Compound of example 2 (PCT/EP2005/051910) was the most potent compound with about 100 % inhibition at 5-10 µM concentration. Compound of Example 2 (PCT/EP2005/051910) was also very potent in inhibiting adhesion in monocytes, while Compound of Example 1 (PCT/EP2005/051908) was ineffective in these cells. Notably, the chemical structure of Compound of Example 1 (PCT/EP2005/051908) makes this compound rather susceptible to esterase action. As monocytes highly express non-specific cytosolic esterases, this likely explains the lack of effect of Compound of Example 1 (PCT/EP2005/051908) on monocyte functions.

Data clearly show that Compound of Example 2 (PCT/EP2005/051910) and Compound of Example 1 (PCT/EP2005/051908) efficiently inhibited chemotaxis in the three major leukocyte sub-types.

Inhibitions observed are clearly concentration-dependent supporting a specific pharmacological effect.

These data suggest that the V-ATPase may be critical target to block the two major cellular events (integrin action and directional motility) responsible of leukocyte recruitment during immune system function and inflammation.

### Effect of V-ATPase inhibitors on spontaneous adhesion of murine encephalitogenic T cells

ICAM-1 and VCAM-1 are adhesion molecules from the family of immunoglobulins and control the migration of lymphocytes through the endothelium during inflammatory responses. Encephalitogenic T cells are activated lymphocytes displaying spontaneous adhesion on ICAM-1 and VCAM-1 and have increased migration capacities in inflammation sites. Spontaneous adhesion of encephalitogenic lymphocytes was measured in adhesion assays on purified ICAM-1 and VCAM-1 as described in the method.

The results on six V-ATPase inhibitors (Table 2) show that treatment of cells with 5 µM of all compounds efficiently blocked spontaneous adhesion on ICAM-1, a ligand for LFA-1 integrin. Compound of Example 2 (PCT/EP2005/051910) was the most potent compound, with an outstanding ability to inhibit the spontaneous adhesion at a concentration of 250 nM. The inhibition observed on ICAM-1 at 5 µM concentration for the remaining compounds was between 47 and 78%, while the inhibition observed using 50 µM concentration was almost complete. Less inhibition was observed with all compounds in adhesion assays on VCAM-1, a ligand for VLA-4 integrin. As shown for ICAM-1, Compound of Example 2 (PCT/EP2005/051910) was the most potent compound. Compound of Example 1 (PCT/EP2005/051908) was also very efficient in blocking spontaneous adhesion to VCAM-1.

These results indicate that some compounds may have integrin-selective blocking effects, while others may have broad effects on integrin family. Inhibition was clearly concentration-dependent in all the experiments supporting a specific pharmacological effect. All treated cells with V-ATPase inhibitors were trypan blue negative and showed no morphological alterations.

### Effect of V-ATPase inhibitors on chemokine-induced adhesion of murine encephalitogenic T cells

Chemokines trigger rapid arrest of lymphocytes in blood vessels through pertussis toxin-sensitive G protein coupled receptors. Rapid adhesion to purified ICAM-1 was measured in in vitro adhesion assays using control/untreated cells and lymphocytes treated with concentration between 5-50 µM for 1 h. Autoreactive lymphocytes were then stimulated with 1 µM CCL19 (MIP-3beta) for 3 min. CCL19 was shown to be expressed on vascular endothelium in lymphoid organs and in sites of inflammation, including inflamed brain endothelium. The results shown in Table 3 demonstrate an almost complete block of autoreactive lymphocyte adhesion with the following compounds: Compound of Example 2 (PCT/EP2005/051910), Compound of Example 1 (PCT/EP2005/051908), Compound of Example 17 (PCT/EP2005/051908), Compound of Example 27 (PCT/EP2005/ 051908), Compound of Example 3 (PCT/EP2005/051908) and Compound of Example 44 (PCT/EP2005/051908). All these compounds were already efficient in blocking adhesion using a concentration of 5 □M for the cell treatment. However, Compound of Example 2 (PCT/EP2005/051910) presented the highest efficiency in blocking activated T cell adhesion; this compound was already extremely efficient at the concentration of 1 µM. Compound of Example 14 (PCT/EP2005/051908) and Compound of Example 76 (PCT/EP2005/051908) blocked adhesion with efficiency between 50-60 % using a concentration of 50 µM (Table 3).

All the other tested compounds inhibited chemokine-induced adhesion with less efficiency or had no significant effect on adhesion at the highest concentration tested (50 µM) when compared with the compounds mentioned above.

Inhibition was clearly concentration-dependent in all the experiments supporting a specific pharmacological effect. Flow cytometry experiments clearly showed that treatment of encephalitogenic T cells with all compounds does not modify the expression of adhesion molecules such as integrins, selectins, mucins and CD44 on cell surface, indicating that V-ATPase inhibitors block adhesion by interfering with signal transduction pathways controlling lymphocyte adhesion. These results, together with the data obtained in spontaneous adhesion assays using encephalitogenic lymphocytes, suggest that V-ATPase may represent critical target to block autoreactive T cell recruitment during autoimmune diseases.

### Effect of V-ATPase inhibitors on NADPH-oxidase activation

The data (Figure 1) clearly show that Compound of Example 2 (PCT/EP2005/051910) and Compound of Example 1 (PCT/EP2005/051908) inhibits NADPH-oxidase activation both in PMNs and in monocytes. Inhibition is concentration-dependent supporting a specific pharmacological effect. Compound of Example 2 (PCT/EP2005/051910) showed a great potency in this model. The data suggest that V-ATPase may be a critical target to block the release of oxygen-derived free radicals, a recognized pro-inflammatory event during the innate immune response.

### In vivo studies

### Effect of a V-ATPase inhibitor on lymphocyte recruitment in inflamed brain microcirculation

In order to verify the effect of Compound of Example 1 (PCT/EP2005/051908) in an experimental model of leukocyte adhesion in vivo, the intravital microscopy experiments in subacutely inflamed brain venules has been performed.

Murine lymphocytes were antigen (PLP139-151 peptide from cerebral proteolipid)-stimulated for 4 days in vitro and then kept for 1-5 days in antigen-free medium prior to intravital microscopy experiments. As expected, treatment of cells with 50 µM of Compound of Example 1 (PCT/EP2005/051908) had no effect on autoreactive lymphocyte rolling. Importantly, sticking/firm arrest was highly blocked by treatment of cells with Compound of Example 1 (PCT/EP2005/051908) (Figure 2). No differences were observed after measuring the hemodynamic parameters (diameter, mean blood flow velocity, wall shear rate, wall shear stress) during administration of control and treated cells, indicating the accuracy of the obtained results. These findings suggest that in an in vivo subacute model of inflammation, V-ATPase play a critical role in the control of lymphocyte migration in inflamed cerebral vessels.

The integrin-dependent leukocyte pro-adhesive activities, including transmigration, protease secretion and inflammatory cytokine-gene expression, play a crucial role in the induction and severity of septic shock. Furthermore, oxygen free radical release has a central role in the peripheral tissue damage related to septic shock and the V-ATPase inhibitors we used are also effective inhibitors of leukocyte NADPH-oxidase, the enzymatic complex responsible of free radical production. Notably, it is quite likely that V-ATPase inhibition is not only effective on leukocytes adhesion; similar molecular events, in fact, control integrin activation also in platelets. For instance, the ras and rho small GTPases control integrin-mediated adhesion and aggregation both in leukocytes as well as in platelets. This is of relevance, as platelets activation and aggregation is an important pathogenetic event during septic shock, and supports that V-ATPase inhibitory drugs can be used to prevent disseminated blood coagulation during septic shock, thus avoiding severe consequences on blood circulation and peripheral tissue perfusion. Thus, inhibition of V-ATPase may globally interfere with molecular mechanisms responsible of activation of pro-inflammatory responses, making V-ATPase inhibitors useful in the therapy of septic shock.

**TABLE 1**

| Compound | Structure | [cpd], µM | Inhibition of Adhesion (%) | | | Inhibition of Chemotaxis (%) | | |
|---|---|---|---|---|---|---|---|---|
| | | | PMNs (fMLP) | Lymphocytes (MIP3-β) | Monocytes (fMLP) | PMNs (fMLP) | Lymphocytes (MIP3-β) | Monocytes (fMLP) |
| Compound of Example 2 (**) | 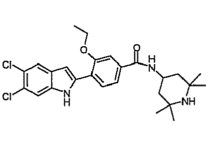 | 1 | 3.4 | 2.9 | 0 | 11.4 | 27.0 | 14.6 |
| | | 2.5 | 71.0 | 13.6 | 86.3 | 53.0 | 46.7 | 44.3 |
| | | 5 | 93.6 | 71.8 | 94.7 | 64.6 | 60.0 | 56.4 |
| | | 10 | 100 | 99.5 | 98.6 | 83.9 | 81.5 | 77.0 |
| | | 25 | 100 | 100 | 100 | 89.7 | 100 | 87.7 |
| | | 50 | 100 | 100 | 100 | 96.3 | 100 | 100 |
| | | 100 | 100 | 100 | 100 | 100 | 100 | 100 |
| | | | | | | | | |
| Compound of Example 1(*) | 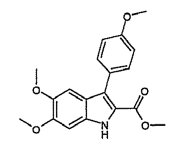 | 2.5 | 9.4 | 32.4 | <5 | 14.6 | 11.6 | 10.5 |
| | | 5 | 56.1 | 41.2 | <5 | 28.2 | 27.2 | 15.5 |
| | | 10 | 66.7 | 42.6 | <5 | 34.0 | 43.2 | 18.3 |
| | | 25 | 72.9 | 60.8 | <5 | 40.6 | 53.3 | 47.4 |
| | | 50 | 79.0 | 61.8 | <5 | 46.4 | 63.5 | 46.3 |
| | | 100 | 94.2 | 98.6 | <5 | 62.6 | 86.9 | 75.5 |
| Compound of Example 14(*) | 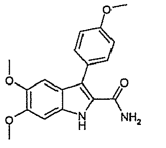 | 25 | 62.8 | 30.9 | | | | |
| | | 50 | 83.9 | 70.9 | | | | |
| | | 100 | 94.9 | 91.8 | | | | |
| Compound of Example26 (*) | 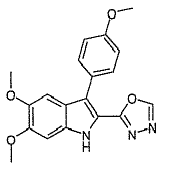 | 25 | 34.7 | 8.7 | | | | |
| | | 50 | 50.5 | 21.9 | | | | |
| | | 100 | 57.5 | 26.7 | | | | |
| Compound of Example 28(*) | 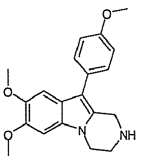 | 25 | 34.7 | 16.7 | | | | |
| | | 50 | 41.0 | 34.8 | | | | |
| | | 100 | 46.5 | 49.1 | | | | |
| Compound of Example39 (*) | 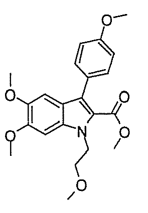 | 25 | 33.8 | 8.2 | | | | |
| | | 50 | 39.1 | 26.4 | | | | |
| | | 100 | 44.8 | 41.4 | | | | |
| Compound of Example 52 (*) | 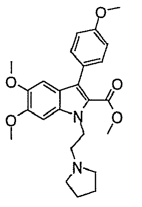 | 25 | 0.8 | 45.9 | | | | |
| | | 50 | 11.0 | 53.5 | | | | |
| | | 100 | 6.1 | 58.9 | | | | |
| Compound of Example 56 (*) | 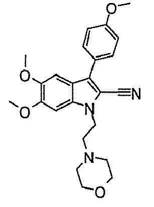 | 25 | 3.4 | 0 | | | | |
| | | 50 | 11.6 | 12.6 | | | | |
| | | 100 | 8.7 | 72.8 | | | | |
| Compound of Example 76 (*) | 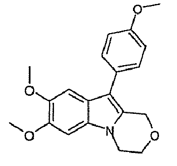 | 25 | 25.6 | 30.1 | | | | |
| | | 50 | 35.7 | 40.3 | | | | |
| | | 100 | 66.0 | 48.3 | | | | |
| Compound of Example 81 (*) | 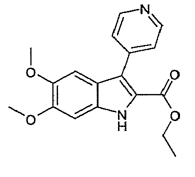 | 25 | 14.4 | 4.5 | | | | |
| | | 50 | 0.8 | 12.7 | | | | |
| | | 100 | 0 | 34.9 | | | | |

**TABLE 2**

| Compound | Structure | [cpd], µM | Inhibition of Spontaneous Adhesion in encephalitogenic lymphocytes (% of control/non-treated cells) | |
|---|---|---|---|---|
| | | | ICAM-1 | VCAM-1 |
| Compound of Example 2 (**) | 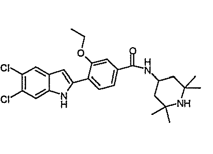 | 0.25 | 83.0 | 11.0 |
| | | 0.5 | 89.2 | 71.0 |
| | | 1 | 95.5 | 99.5 |
| | | 5 | 96.6 | 100 |
| | | 10 | 99.7 | 100 |
| Compound of Example 1 (*) | 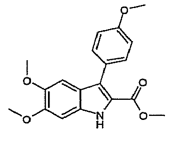 | 5 | 47.0 | 46.0 |
| | | 10 | 65.0 | 66.0 |
| | | 25 | 70.0 | 74.0 |
| | | 50 | 89.0 | 92.4 |
| | | 100 | 96.0 | n.d. |
| Compound of Example 17 (*) | 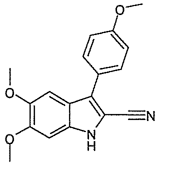 | 5 | 72.8 | 34.0 |
| | | 10 | 72.5 | 53.0 |
| | | 25 | 76.0 | 71.5 |
| | | 50 | 92.0 | 89.0 |
| | | 100 | 99.0 | n.d. |
| Compound of Example 27 (*) | 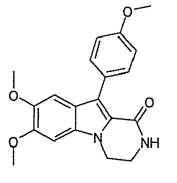 | 5 | 69.0 | 11.0 |
| | | 10 | 76.0 | 31.0 |
| | | 25 | 82.0 | 35.5 |
| | | 50 | 90.0 | 82.0 |
| | | 100 | 99.0 | n.d. |
| Compound of Example 3 (*) | 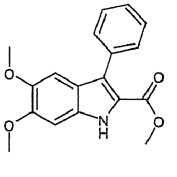 | 5 | 78.0 | 6.0 |
| | | 10 | 79.5 | 9.0 |
| | | 25 | 90.0 | 32.5 |
| | | 50 | 92.0 | 71.4 |
| | | 100 | 99.0 | n.d. |
| Compound of Example 44 (*) | 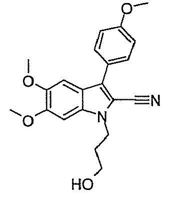 | 5 | 75.0 | 29.0 |
| | | 10 | 84.0 | 39.0 |
| | | 25 | 88.0 | 43.0 |
| | | 50 | 91.5 | 84.0 |
| | | 100 | 99.0 | n.d. |

**TABLE 3**

| Compound | Structure | [cpd], µM | Chemokine-induced Adhesion in encephalitogenic lymphocytes Mean counts ± S.D. | |
|---|---|---|---|---|
| | | | in absence of MIP3-β stimulation | in presence of MIP3-β stimulation |
| Compound of Example 2 (**) | 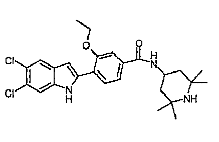 | NT | 29.0 ± 8.0 | 68.0 ± 7.0 |
| | | 1 | 1.3 ± 2.8 | 11.4 ± 5.4 |
| | | 5 | 0.6 ± 1.6 | 4.6 ± 2.4 |
| | | 10 | 0.4 ± 0.4 | 0.4 ± 0.8 |
| Compound of Example 1 (*) | 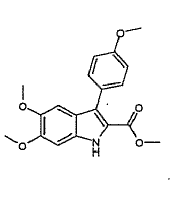 | NT | 29.0 ± 8.0 | 68.0 ± 7.0 |
| | | 5 | 1.3 ± 1.0 | 10.3 ± 1.0 |
| | | 1 0 | 1.0 ± 1.0 | 7.0 ± 1.0 |
| | | 25 | 0.4 ± 0.2 | 5.3 ± 2.0 |
| | | 50 | 0.4 ± 0.1 | 3.2 ± 1.0 |
| Compound of Example 17 (*) | 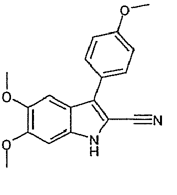 | NT | 29.0 ± 8.0 | 68.0 ± 7.0 |
| | | 5 | 2.0 ± 1.0 | 13.6 ± 3.5 |
| | | 10 | 0.9 ± 0.2 | 10.0 ± 3.0 |
| | | 25 | 0.6 ± 0.5 | 1.8 ± 1.0 |
| | | 50 | 0.4 ± 0.1 | 2.1 ± 0.9 |
| Compound of Example 14 (*) | 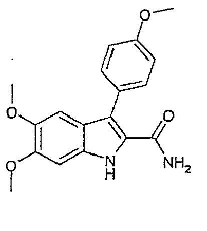 | NT | 90.9 ± 5.3 | 140.0 ± 3.6 |
| | | 1 | 67.6 ± 2.0 | 120.3 ± 9.8 |
| | | 5 | 41.8 ± 4.2 | 101.2 ± 6.1 |
| | | 50 | 20.9 ± 11.7 | 56.8 ± 2.2 |
| Compound of Example 26 (*) | 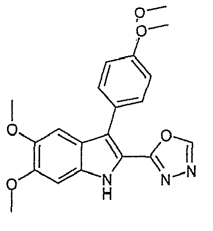 | NT | 90.9 ± 5.3 | 140.0 ± 3.6 |
| | | 5 | 93.1 ± 7.5 | 125.9 ± 7.2 |
| | | 50 | 49.8 ± 5.9 | 98.9 ± 12.0 |
| Compound of Example 27 (*) | 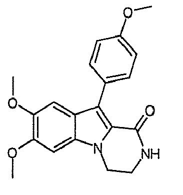 | NT | 29.0 ± 8.0 | 68.0 ± 7.0 |
| | | 5 | 5.0 ± 2.2 | 17.7 ± 2.5 |
| | | 10 | 1.7 ± 1.0 | 18.1 ± 4.3 |
| | | 25 | 1.5 ± 0.1 | 9.3 ± 0.5 |
| | | 50 | 1.2 ± 1.1 | 5.1 ± 1.1 |
| Compound of Example 3 (*) | 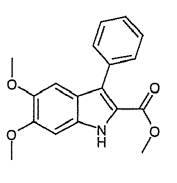 | NT | 29.0 ± 8.0 | 68.0 ± 7.0 |
| | | 5 | 1.5 ± .2.0 | 10.5 ± 3.0 |
| | | 10 | 1.8 ± 1.0 | 8.7 ± 3.0 |
| | | 25 | 1.2 ± 1.0 | 3.5 ± 1.5 |
| | | 50 | 0.5 ± 2.0 | 5.0 ± 2.0 |
| Compound of Example 28 (*) | 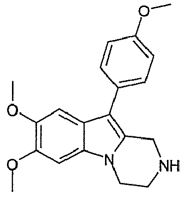 | NT | 90.9 ± 5.3 | 140.0 ± 3.6 |
| | | 5 | 75.7 ± 9.0 | 127.0 ± 5.0 |
| | | 50 | 48.6 ± 2.9 | 118.8 ± 10.8 |
| Compound of Example 39 (*) | 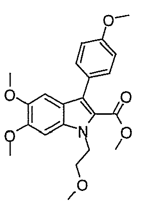 | NT | 90.9 ± 5.3 | 140.0 ± 3.6 |
| | | 5 | 72.9 ± 8.8 | 118.2 ± 10.0 |
| | | 50 | 44.8 ± 1.9 | 104.4 ± 5.0 |
| Compound of Example 50 (*) | 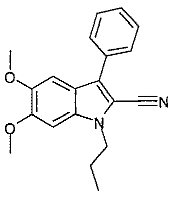 | NT | 90.9 ± 5.3 | 140.0 ± 3.6 |
| | | 5 | 81.7 ± 11.9 | 124.5 ± 8.1 |
| | | 50 | 62.0 ± 4.0 | 92.0 ± 3.0 |
| Compound of Example 52 (*) | 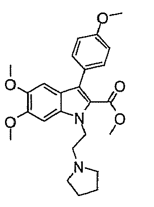 | NT | 90.9 ± 5.3 | 140.0 ± 3.6 |
| | | 1 | 51.9 ± 3.9 | 118.9 ± 4.0 |
| | | 5 | 31.8 ± 3.1 | 103.2 ± 10.2 |
| | | 50 | 24.9 ± 12.8 | 82.3 ± 8.1 |
| Compound of Example 44 (*) | 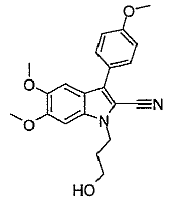 | NT | 29.0 ± 8.0 | 68.0 ± 7.0 |
| | | 5 | 1.0 ± 0.9 | 2.1 ± 0.5 |
| | | 10 | 0.4 ± 1.7 | 3.7 ± 1.1 |
| | | 25 | 0.4 ± 0.1 | 1.8 ± 0.5 |
| | | 50 | 0.4 ± 0.5 | 0.1 ± 0.5 |
| Compound of Example 56 (*) | 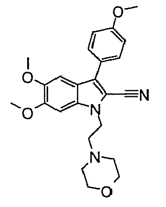 | NT | 90.9 ± 5.3 | 140.0 ± 3.6 |
| | | 5 | 64.1 ± 9.9 | 92.3 ± 2.3 |
| | | 50 | 49.7 ± 3.9 | 79.4 ± 12.1 |
| Compound of Example 76 (*) | 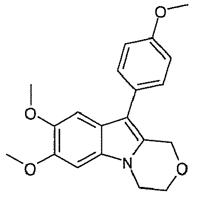 | NT | 90.9 ± 5.3 | 140.0 ± 3.6 |
| | | 1 | 36.9 ± 4.0 | 102.9 ± 2.9 |
| | | 5 | 28.3 ± 8.9 | 87.7 ± 12.0 |
| | | 50 | 23.0 ± 1.9 | 66.6 ± 1.8 |
| Compound of Example 68 (*) | 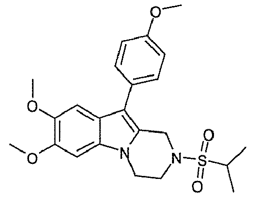 | NT | 90.9 ± 5.3 | 140.0 ± 3.6 |
| | | 5 | 62.3 ± 9.0 | 102.8 ± 5.5 |
| | | 50 | 33.5 ± 6.0 | 74.3 ± 3.9 |
| Compound of Example 81 (*) | 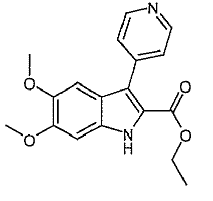 | NT | 90.9 ± 5.3 | 140.0 ± 3.6 |
| | | 5 | 56.2 ± 11.1 | 84.5 ± 8.8 |
| | | 50 | 41.3 ± 3.6 | 99.2 ± 5.0 |

| | | | | |
|---|---|---|---|---|
| (*): PCT/EP2005/051908 (**): PCT/EP2005/051910 | | | | |

## Claims

1. Use of one or more V-ATPase inhibitors in the preparation of a medicament useful for preventing and/or treating septic shock.

2. Use according to claim 1, wherein said V-ATPase inhibitor is selected from bafilomycins, concanamycins, depsipeptide mycotoxins derived from the fungi *Metharisium anisopliae,* destruxin, lobatamides, salicylihalamides, oximidines, and derivatives thereof.

3. Use according to claim 1, wherein said V-ATPase inhibitor is selected from sulphonamide derivatives of bafilomycins, 7,21-0-disubstituted bafilomycins, 2-methoxy-2,4-pentadienoic esters of bafilomycins.

4. Use according to claim 1 wherein said V-ATPase inhibitor is selected from N-ethylmaleimide, 7-chloro-4-nitrobenzo-2-oxa-1,3-diazole, WY 47766; SB242784, FR167356.

5. Use according to claim 1 wherein said V-ATPase inhibitor is a 2- and/or 3-substituted indole, a 2- and/or 3- substituted azaindole or a 2- and/or 3-substituted benzimidazole.

6. Use according to claims 1-5, wherein said V-ATPase inhibitor is administered in the form of pharmaceutical compositions in the presence of suitable pharmaceutical excipients and possible additional drugs useful in septic shock therapy.

7. Use according to claim 6, wherein said pharmaceutical composition contain said V-ATPase inhibitor in a quantity between 1 and 1000 mg.

8. Use according to claims 7, wherein said composition is adapted to deliver to the patient a dosage comprised between 0.01 and 100 mg/Kg.

9. Use according to claims 6-8, wherein said pharmaceutical composition is suitable for oral, buccal, intravenous, intramuscular, intradermic, transdermal, topic, ophthalmic, intraauricular or inhalatory administration route.

10. Use according to claims 6-9, wherein said pharmaceutical composition is provided in the form of injectable solutions, solutions for infusion, solutions for inhalation, suspensions, emulsions, syrups, elixirs, drops, suppositories, possibly coated pills, hard or soft capsules, microcapsules, granules, dispersible powders.
